Europäisches Patentamt

European Patent Office

Office européen des brevets

(11) Veröffentlichungsnummer: **0 261 598**
**B1**

(12) # EUROPÄISCHE PATENTSCHRIFT

(45) Veröffentlichungstag der Patentschrift:
**20.06.90**

(51) Int. Cl.⁵: **A61M 37/00**

(21) Anmeldenummer: **87113664.4**

(22) Anmeldetag: **18.09.87**

(54) Injektionseinrichtung.

(30) Priorität: **22.09.86 DE 8625575 U**
**22.09.86 DE 8625574 U**

(43) Veröffentlichungstag der Anmeldung:
**30.03.88 Patentblatt 88/13**

(45) Bekanntmachung des Hinweises auf die Patenterteilung:
**20.06.90 Patentblatt 90/25**

(84) Benannte Vertragsstaaten:
**AT BE CH DE ES FR GB GR IT LI LU NL SE**

(56) Entgegenhaltungen:
**DE-A- 3 035 009**
**FR-A- 1 133 709**
**FR-A- 1 142 769**
**US-A- 3 964 482**
**US-A- 4 159 720**

(73) Patentinhaber: **SCHERING AKTIENGESELLSCHAFT**
**Berlin und Bergkamen,**
**Müllerstrasse 170/178 Postfach 65 03 11,**
**D-1000 Berlin 65(DE)**

(72) Erfinder: **Przuntek, Horst, Prof. Dr. med.,**
**Schinkelstrasse 46, D-4630 Bochum(DE)**
Erfinder: **Bittkau, Simon, Dr. Neurologische**
**Universitäts-, Klinik Würzburg**
**Josef-Schneider-Strasse 11, D-8700 Würzburg(DE)**

(74) Vertreter: **Maikowski, Michael, Dipl.-Ing. Dr.,**
**Patentanwälte Maikowski & Ninnemann Xantener**
**Strasse 10, D-1000 Berlin 15(DE)**

ACTORUM AG

## Beschreibung

Die Erfindung betrifft eine Injektionseinrichtung nach dem Oberbegriff des Anspruchs 1.

Es ist bekannt, Injektionsnadeln zum kontinuierlichen oder periodischen subkutanen oder intramuskulären Einbringen einer wässrigen oder öligen Heilmittellösung im Körpergewebe zu verwenden. Diese Injektionsnadeln können mittels eines Schlauches aus gegenüber der Heilmittellösung indeferentem und gewerbeverträglichem Material mit einer mechanischen oder automatischen Dosiereinrichtung verbunden sein.

Zur Behandlung bestimmter Krankheiten wie beispielsweise des Morbus Parkinson ist eine kontinuierliche oder periodische Zufuhr der Heilmittellösung erwünscht, da nur auf diese Weise ein gleichmäßiger Wirkstoffspiegel im Blut gewährleistet werden kann. Um dem Patienten sowohl die Bewegungsfreiheit zu erhalten, als auch eine stationäre Behandlung und Einnahmefehler zu vermeiden, verwendet man dazu üblicherweise eine elektrische Dosierpumpe, die am Körper getragen wird und die das Heilmittel über eine Schlauchleitung durch eine im subcutanen oder Muskelgewebe fixierte Injektionskanüle abgibt. Dabei treten naturgemäß an der Eintrittsstelle im Körpergewebe höhere Heilmittelkonzentrationen als benötigt auf. Die am Injektionsort auftretende erhöhte Heilmittelkonzentration kann in Abhängigkeit von der chemischen Natur des Heilmittels sowie dessen Konzentration zu örtlichen Reizerscheinungen und entzündungsähnlichen Zuständen führen, die im Extremfall die Beendigung der Therapie erforderlich machen.

In der US-PS 4 159 720 wird eine Vorrichtung beschrieben, die die Heilmittellösung aus einem auf der Haut befestigten Vorratsbehältnis durch einen im Gewebe befindlichen Docht aus Seide oder medizinischem Nahtmaterial in den Körper bringt. Diese Methode hat den Nachteil, daß die Dosierung durch die Docht-Oberfläche vorgegeben und konstant ist, somit keine dem Tagesprofil angepaßte Dosierung möglich ist. Darüber hinaus muß die Gewebeverträglichkeit des Dochtmaterials im speziellen Fall sichergestellt sein.

In der DE-OS 3 035 009 wird ein Spritzenkopf beschrieben, der mit 5 Stutzen zur Aufnahme von Injektionsnadeln versehen ist. Dieser ist jedoch als Teil einer Injektionsspritze zur Quaddelbildung vorgesehen. Bedingt durch die räumliche Ausdehnung und die konstruktiven Einzelheiten des Spritzenkopfes und der Stutzen zur Aufnahme der Injektionsspritzen ist diese Vorrichtung nicht geeignet, um nach Fixierung am gewünschten Injektionsort längere Zeit am Körper getragen zu werden.

In der FR-PS 1 142 769 wird eine Injektionsnadel beschrieben, die seitliche Öffnungen aufweist, so daß das zu injizierende Medikament im Gewebe längs der Nadel verteilt wird. Dies kann trotz der Verteilung in Tiefenwirkung zu unerwünschten Konzentrationen an der Einstichstelle führen.

Die FR-PS 1 133 709 beschreibt ein Gerät zur Reflextherapie, das einen kastenförmigen Vorratsbehälter für ein Medikament aufweist, von dessen Boden sich eine Anzahl paralleler Injektionsnadeln erstrecken, die mit dem Vorratsbehälter über Kapillaren verbunden sind, so daß nur eine Einbringung von Tropfen im Hautbereich erfolgt. Dieses Gerät ist für eine Fixierung am Körper für längere Zeit nicht geeignet.

Von diesem Stand der Technik ausgehend, liegt der Erfindung die Aufgabe zugrunde, eine Injektionseinrichtung für eine Dauerinjektion zu schaffen, die zur Behandlung verschiedener Krankheiten am Körper leicht fixiert und gespeist werden kann, damit durch Vergrößerung der Injektionsfläche die Konzentration der Heilmittellösung am Eintrittsort bei gleichbleibender Zuflußgeschwindigkeit vermindert werden kann. So wird die durch hohe Heilmittelkonzentration am Eintrittsort hervorgerufene Gewebe-Reizung und Entzündung vermieden.

Erfindungsgemäß wird diese Aufgabe durch die technische Lehre des Anspruchs 1 gelöst.

Die Injektionseinrichtung ermöglicht eine vorbestimmte flächenhafte Verteilung der zu injizierenden Lösung. Die Halterung ist insbesondere so flach gestaltet, daß sie am Injektionsort ohne großen Raumbedarf gut fixierbar ist, beispielsweise flach am Körper aufliegt und im einfacher Weise gespeist werden kann. Die Injektionseinrichtung kann durch die parallelen Längsachsen der Kanülen in einfacher Weise appliziert werden. Ein einfacher Aufbau wird dadurch erzielt, daß eine die starre Halterung und Speiseleitung bildende Rohrleitungsanordnung vorgesehen ist, bei der die Rohrachse oder die Rohrachsen in einer Ebene liegen und mit der die Kanülen, die auf einer Seite dieser Ebene senkrecht zu dieser angeordnet sind, verbunden sind. Mit besonderem Vorteil ist die Rohrleitungsanordnung ein Rohrleitungsring. Ein derartiger Rohrleitungsring erfüllt in idealer Weise die Erfordernisse gut fixierbar zu sein und ohne großen Raumbedarf flach am Körper aufzuliegen. Ein tangentialer Anschlußstutzen ist für diesen Rohrleitungsring vorgesehen. Insbesondere sind die Kanülen im gleichen Abstand voneinander angeordnet.

Falls neben der flächenhaften Verteilung noch eine zusätzliche Verteilung in die Tiefe erwünscht ist, sind die Kanülen mit einem geschlossenen Kanülenanschliff und mehreren seitlichen Austrittsöffnungen versehen.

Um die Applikation der erfindungsgemäßen Injektionseinrichtung durch den Patienten selbst zu erleichtern, kann in vorteilhafter Weise an der die Kanülen tragenden starren Halterung ein Griff z.B. in Bogenform angeordnet sein.

Ein Ausführungsbeispiel der Erfindung soll unter Bezugnahme auf die Figuren der Zeichnung beschrieben werden:

Es zeigen:

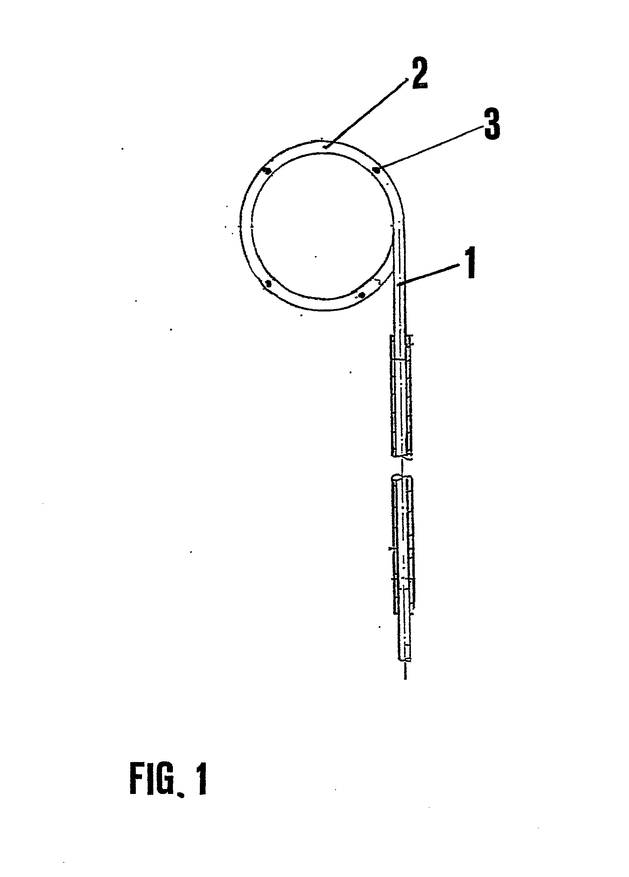

Fig. 1 eine Draufsicht auf einen Rohrleitungsring

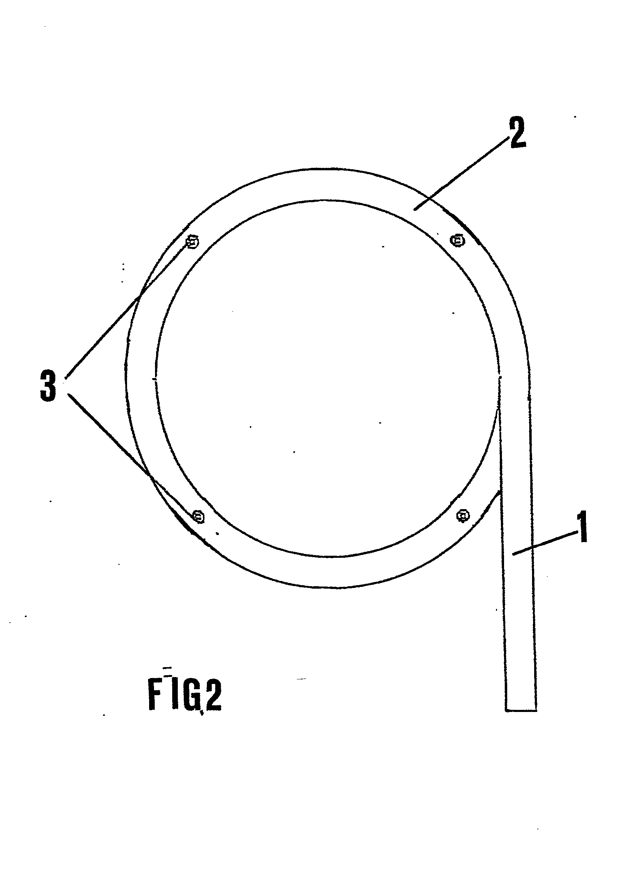

Fig. 2 eine vergrößerte Ansicht des Rohrleitungsringes von unten
und
Fig. 3 eine schematische, teilweise geschnittene Seitenansicht.

In den Figuren ist ein Rohrleitungsring 2 dargestellt. Dieser Rohrleitungsring 2 weist einen tangentialen Anschlußstutzen 1 auf, der beispielsweise mit einem Teflonschlauch in Verbindung stehen kann, an den sich ein Luer-Lookanschluß anschließt.

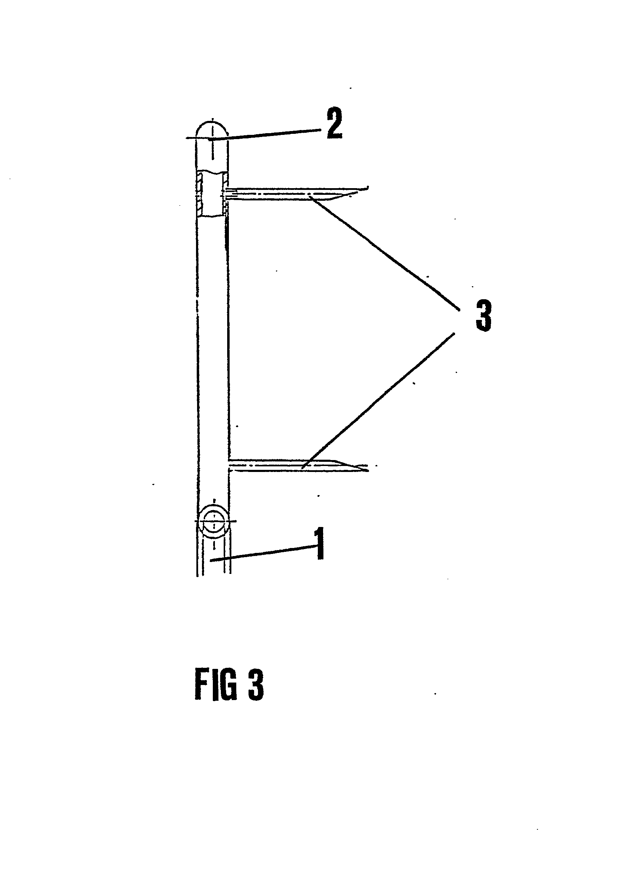

Wie insbesondere Fig. 3 zeigt, erstrecken sich senkrecht zur Ebene A–A des Rohrleitungsringes 2 auf einer Seite mehrere Kanülen 3, die mit diesem Rohrleitungsring in Verbindung stehen. Die Achsen der Kanülen 3 verlaufen parallel zueinander.

Eine bevorzugte Ausführungsform besteht darin, daß der zylindrische Hohlkörper ein ringförmiges Rohr mit einem Außendurchmesser von 20 mm darstellt. Der Rohrinnendurchmesser beträgt 1 mm, der Rohraußendurchmesser 1,5 mm. Senkrecht zur Ringebene sind auf derselben Seite parallel und im gleichen Abstand zueinander 4 Injektionskanülen mit einer lichten Weite von 0,2 mm und einem Außendurchmesser von 0,45 mm angebracht. Das ringförmige Rohr besitzt außerdem tangential und parallel zur Ringebene ein gerades Anschlußrohr von 16 mm Länge mit demselben Rohrquerschnitt wie das ringförmige Rohr. Durch die Anordnung der Injektionskanülen auf einem ringförmigen Rohr besitzt außerdem tangential und parallel zur Ringebene ein gerades Anschlußrohr von 16 mm Länge mit demselben Rohrquerschnitt wie das ringförmige Rohr. Durch die Anordnung der Injektionskanülen auf einem ringförmigen Rohr läßt sich diese Injektionspritze nach dem Einstich der Kanülen am geeigneten Injektionsort durch geeignete Maßnahmen (physiologisch verträglicher Kreuz-Plaster-Verband o.ä.) gut am Körper fixieren.

Die Handhabung der erfindungsgemäßen Injektionsnadel ist wie folgt:

Das gesamte System (Dosiereinrichtung, Zuführungsschlauch und Injektionseinrichtung) wird blasenfrei mit der Heilmittellösung gefüllt und die Spritze so an der gewünschten Stelle in das Körpergewebe eingestochen, daß das ringförmige Rohr auf der Haut aufliegt. Die Injektionseinrichtung wird nun durch ein physiologisch verträgliches Pflaster o.ä. am Körper befestigt und die Dosiereinrichtung in Betrieb gesetzt.

**Patentansprüche**

1. Injektionseinrichtung mit mehreren, in einer starren Halterung im Abstand voneinander gehaltenen mit einem Vorrat verbundenen Kanülen (3), deren Längsachsen parallel zueinander angeordnet sind gekennzeichnet durch eine, die starre Halterung und eine Speiseleitung bildende Rohrleitungsanordnung (2), deren Rohrachse oder Rohrachsen in einer Ebene liegen, wobei die Kanülen (3) mit der Rohrleitungsanordnung (2) und auf einer Seite der Ebene senkrecht zu dieser angeordnet sind.

2. Injektionseinrichtung nach Anspruch 1, dadurch gekennzeichnet, daß die Rohrleitungsanordnung ein Rohrleitungsring (2) ist.

3. Injektionseinrichtung nach Anspruch 2 gekennzeichnet durch einen tangentialen Anschlußstutzen (1) für den Rohrleitungsring (2).

4. Injektionseinrichtung nach mindestens einem der Ansprüche 1–3, dadurch gekennzeichnet, daß die Kanülen (3) im gleichen Abstand voneinander angeordnet sind.

5. Injektionseinrichtung nach mindestens einem der Ansprüche 1–4, dadurch gekennzeichnet, daß die Kanülen einen geschlossenen Kanülenanschliff und mehrere seitliche Austrittsöffnungen aufweisen.

6. Injektionseinrichtung nach mindestens einem der Ansprüche 1–5, dadurch gekennzeichnet, daß an der starren Halterung ein Griff angeordnet ist.

**Claims**

1. Injection device with several cannulae (3) held spaced apart from one another in a rigid mounting support and connected with a supply duct, the longitudinal axes of which cannulae are arranged parallel to one another, characterised by a tubular duct arrangement (2) forming the rigid mounting support and supply duct, the tubular axis or tubular axes of the duct arrangement lying in a plane so that the cannulae (3) are connected with the tubular duct arrangement and are arranged on one side of the plane and perpendicular to it.

2. Injection device according to Claim 1, characterised in that the tubular duct arrangement is a tubular duct ring (2).

3. Injection device according to Claim 2 characterised by a tangential connection spigot (1) for the tubular duct ring (2).

4. Injection device according to at least one of the Claims 1 to 3, characterised in that the cannulae (3) are arranged equally spaced from one another.

5. Injection device according to at least one of the Claims 1 to 4, characterised in that the cannulae have a closed cannula tip and several lateral exit openings.

6. Injection device according to at least one of Claims 1 to 5, characterised in that a handle is mounted on the rigid holder.

**Revendications**

1. Dispositif d'injection comportant plusieurs canules (3), reliées à un réservoir, tenues distantes les unes des autres dans une fixation rigide, les axes desdites canules étant disposés de manière parallèle les uns par rapport aux autres, caractérisé par un agencement de tuyaux (2) formant la fixation rigide et la conduite d'alimentation, dans lequel l'axe du tuyau ou les axes des tuyaux se trouvent dans un plan, les canules (3) étant reliées à l'agencement de tuyaux (2) et disposées d'un côté de ce plan perpendiculairement à celui-ci.

2. Dispositif d'injection selon la revendication 1, caractérisé en ce que l'agencement de tuyaux est un anneau tubulaire (2).

3. Dispositif d'injection selon la revendication 2, caractérisé par une tubulure de raccordement (1) tangentielle pour l'anneau tubulaire (2).

4. Dispositif d'injection selon au moins l'une des revendications 1 à 3, caractérisé en ce que les canules (3) sont disposées à une distance identique les unes des autres.

5. Dispositif d'injection selon au moins l'une des revendications 1 à 4, caractérisé en ce que les canules présentent un affûtage de canule fermé et plusieurs ouvertures de sortie latérales.

6. Dispositif d'injection selon au moins l'une des revendications 1 à 5, caractérisé en ce qu'une poignée est agencée sur la fixation rigide.

**2**

**3**

**1**

FIG. 1

FIG 2

**FIG 3**